(19) Europäisches Patentamt
European Patent Office
Office européen des brevets

(11) EP 1 559 413 A2

(12) # EUROPÄISCHE PATENTANMELDUNG

(43) Veröffentlichungstag:
**03.08.2005 Patentblatt 2005/31**

(51) Int Cl.[7]: **A61K 7/42**

(21) Anmeldenummer: **05005833.8**

(22) Anmeldetag: **01.04.1999**

(84) Benannte Vertragsstaaten:
**DE ES FR GB IT SE**

(30) Priorität: **18.04.1998 DE 19817292**

(62) Dokumentnummer(n) der früheren Anmeldung(en) nach Art. 76 EPÜ:
**99106119.3 / 0 950 396**

(71) Anmelder: **Beiersdorf AG**
**20253 Hamburg (DE)**

(72) Erfinder: **Die Erfindernennung liegt noch nicht vor**

Bemerkungen:
Diese Anmeldung ist am 17 - 03 - 2005 als Teilanmeldung zu der unter INID-Kode 62 erwähnten Anmeldung eingereicht worden.

(54) **Stabile kosmetische oder dermatologische Zubereitungen in Form von W/O-Emulsionen enthaltend unsymmetrisch substituierte Triazinderivate**

(57) Kosmetische oder dermatologische Lichtschutzzubereitungen in Form von W/O-Emulsionen, enthaltend Wirkstoffkombinationen aus

(a) einem oder mehreren unsymmetrisch substituierten s-Triazinderivaten
(b) einer oder mehreren grenzflächenaktiven Substanzen

EP 1 559 413 A2

## Beschreibung

Die vorliegende Erfindung betrifft kosmetische und dermatologische Zubereitungen mit einem Gehalt an unsymmetrisch substituierten Triazinderivaten. Die vorliegende Erfindung betrifft kosmetische und dermatologische Lichtschutzzubereitungen, insbesondere hautpflegende kosmetische und dermatologische Lichtschutzzubereitungen. In einer besonders bevorzugten Ausführungsform betrifft die vorliegende Erfindung wasserfeste Lichtschutzzubereitungen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen der Haut.

Als ein Maximum der Erythemwirksamkeit des Sonnenlichtes wird der engere Bereich um 308 nm angesehen.

Zum Schutze gegen UVB-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Wellenlängenbereich zwischen etwa 320 und 400 nm, den sogenannten UVA-Bereich, sind UV-Filtersubstanzen wichtig, da auch solche Strahlen Schäden hervorrufen können. So ist erwiesen, daß UVA-Strahlung zu einer Schädigung der elastischen und kollagenen Fasern des Bindegewebes führt, was die Haut vorzeitig altern läßt und als Ursache zahlreicher phototoxischer und photoallergischer Reaktionen zu sehen ist. Der schädigende Einfluß der UVB-Strahlung kann durch UVA-Strahlung verstärkt werden.

Die UV-Strahlung kann aber auch zu photochemischen Reaktionen führen, wobei dann die photochemischen Reaktionsprodukte in den Haut- und Zellmetabolismus eingreifen.

Vorwiegend handelt es sich bei solchen photochemischen Reaktionsprodukten um radikalische Verbindungen, z.B. Hydroxyradikale, Hydroperoxyradikale sowie Superoxidionen. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls, kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Um diesen Reaktionen vorzubeugen, können den kosmetischen bzw. dermatologischen Formulierungen zusätzlich Antioxidantien und/oder Radikalfänger einverleibt werden.

UV-Absorber bzw. UV-Reflektoren sind die meisten anorganischen Pigmente, die bekannterweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen.

Ganz besonders nachteilig an vielen Wasser-in-Öl-Emulsionen des Standes der Technik ist ferner, daß sie nicht stabil gegen physikalische Zersetzung sind. Üblicherweise bezeichnet man das am häufigsten auftretende unerwünschte Verhalten als "Ausölen". Dies besagt, daß sich Wasserphase und Ölphase allmählich trennen. Dies äußert sich beispielsweise darin, daß aus einer W/O-Creme (= Wasser-in-Öl-Crème) Öl austritt. Beginnt aber eine Emulsion erst einmal, sich zu zersetzen, so ist dieser Vorgang nicht mit einfachen Mitteln wieder rückgängig zu machen. Zumindest stehen dem Verbraucher solcher Mittel nicht zur Verfügung.

Aufgabe der vorliegenden Erfindung war es also auch, die Nachteile des Standes der Technik in dieser Hinsicht zu beseitigen. Insbesondere sollten stabile hautpflegende kosmetische Mittel zur Verfügung gestellt werden.

Von verschiedenen Autoren wurden UV-Filtersubstanzen vorgestellt, welche das Strukturmotiv

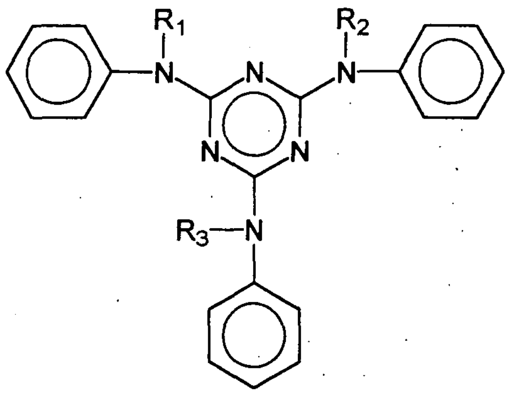

aufweisen.

Hinsichtlich der $C_3$-Achse des Triazingrundkörpers sind sowohl symmetrische Substitution wie auch unsymmetrische Substitution denkbar. In diesem Sinne symmetrisch substituierte s-Triazine weisen drei gleiche Substituenten auf und werden beispielsweise vertreten durch den 4,4',4"-(1,3,5-Triazin-2,4,6-triyltriimino)-tris-benzoesäure-tris (2-ethylhexylester), analog der INCI-Nomenklatur auch: Octyltriazon, welcher durch folgende Struktur wiedergegeben wird:

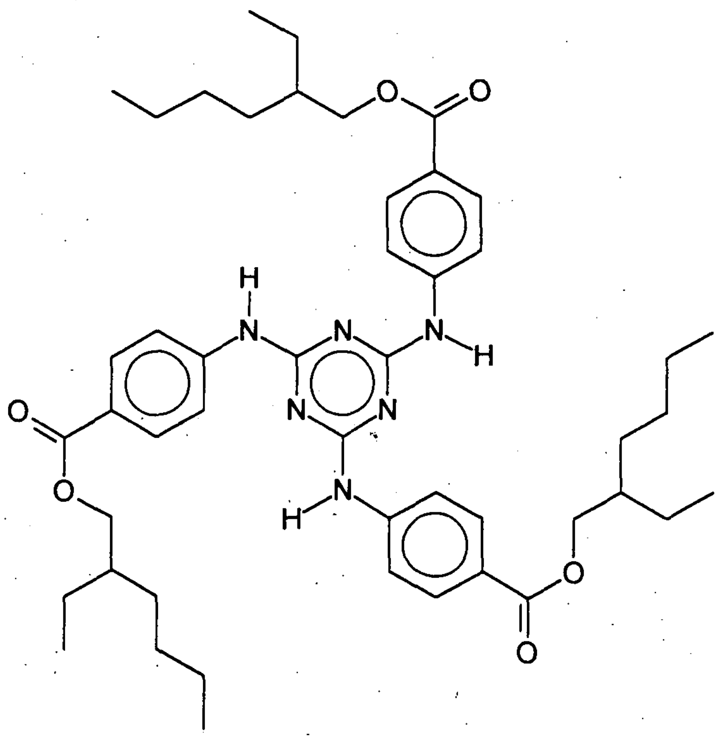

Hinsichtlich der $C_3$-Achse unsymmetrisch substituierte s-Triazinderivate weisen demzufolge unterschiedliche Substituenten auf, wodurch die $C_3$-Symmetrie zerstört wird. Im Sinne der hiermit vorliegenden Erfindung wird als "symmetrisch" bzw. "unsymmetrisch" stets symmetrisch bzw. unsymmetrisch hinsichtlich der $C_3$-Achse des Triazingrundkörpers verstanden, es sei denn, etwas Anderes wäre ausdrücklich erwähnt.

So werden in der EP-A- 570 838 unsymmetrisch substituierte s-Triazinderivate beschrieben, deren chemische Struktur durch die generische Formel

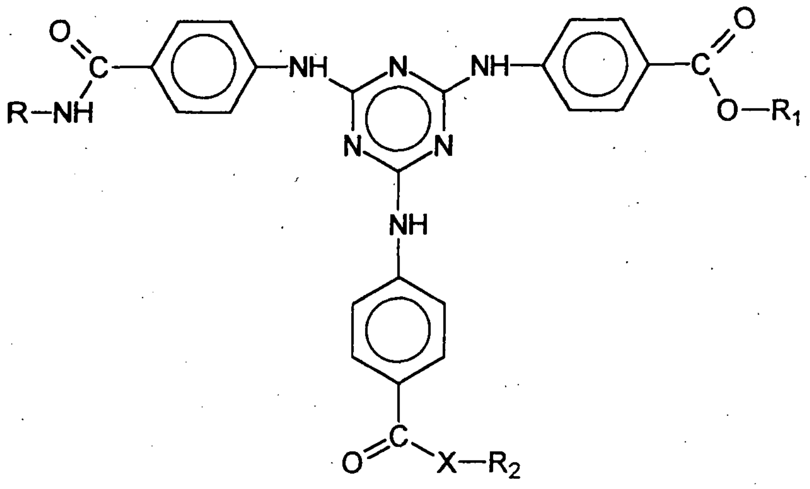

wiedergegeben wird, wobei

R einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt,

X ein Sauerstoffatom oder eine NH-Gruppe darstellt,

$R_1$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

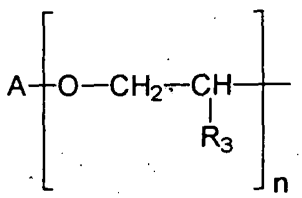

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,

$R_3$ ein Wasserstoffatom oder eine Methylgruppe darstellt,

n eine Zahl von 1 bis 10 darstellt,

$R_2$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt, wenn X die NH-Gruppe darstellt, und
einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

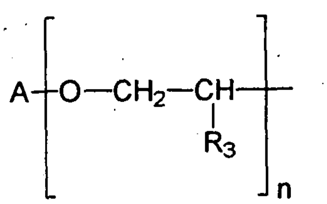

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,
$R_3$ ein Wasserstoffatom oder eine Methylgruppe darstellt,
n eine Zahl von 1 bis 10 darstellt,

wenn X ein Sauerstoffatom darstellt.

Solche s-Triazinderivate, die sich im Gegensatze zum 4,4',4"-(1,3,5-Triazin-2,4,6-triyltriimino)-tris-benzoesäure-tris(2-ethylhexylester) durch verbesserte Löslichkeit in vielen Ölkomponenten auszeichnen, können gemäß der Lehre der Schriften EP-A-821 937, EP-A-821 938, EP-A-821 939, EP-A-821 940 und EP-A-821 941 mit verschiedenen anderen Lichtschutzfiltern in kosmetischen oder dermatologischen Zubereitungen kombiniert werden, wodurch bestimmten technischen Sachverhalten Rechnung getragen werden soll.

Eine Aufgabe der vorliegenden Erfindung war es also, all diesen Übelständen Abhilfe zu schaffen. Weiterhin war eine Aufgabe der vorliegenden Erfindung, Lichtschutzzubereitungen zur Verfügung zu stellen, die sich durch eine hohe Lichtschutzwirkung und gute kosmetische und dermatologische Akzeptanz auszeichnen.

Erstaunlicherweise werden alle diese Aufgaben gelöst durch kosmetische oder dermatologische Lichtschutzzubereitungen enthaltend Wirkstoffkombinationen aus

(a) einem oder mehreren unsymmetrisch substituierten s-Triazinderivaten
(b) einer oder mehreren grenzflächenaktiven Substanzen der der allgemeinen Formel (1)

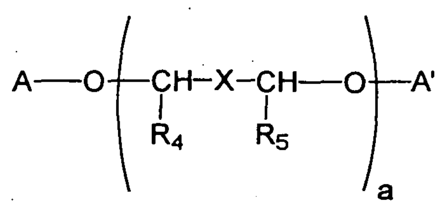

,

- wobei A und A' gleiche oder verschiedene organische Reste, gewählt aus der Gruppe der verzweigten und unverzweigten, gesättigten und ungesättigten Alkyl- und Acylreste und Hydroxyacylreste mit 10 - 30 Kohlenstoffatomen sowie ferner aus der Gruppe der über Esterfunktionen miteinander verbundenen Hydroxyacylgruppen, nach dem Schema

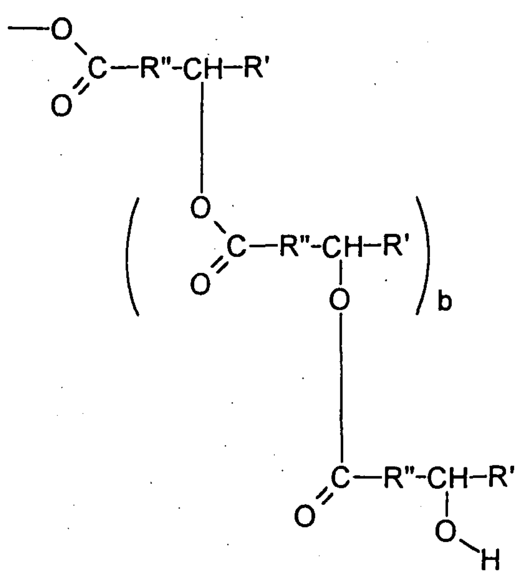

,

wobei R' gewählt wird aus der Gruppe der verzweigten und unverzweigten Alkylgruppen mit 1 bis 20 Kohlenstoffatomen und R" gewählt wird aus der Gruppe der verzweigten und unverzweigten Alkylengruppen mit 1 bis 20 Kohlenstoffatomen und b Zahlen von 0 bis 200 annehmen kann,

- a eine Zahl von 1 bis 100, vorzugsweise 2 bis 60, insbesondere 5 bis 40 darstellt,
- X eine Einfachbindung oder die Gruppe

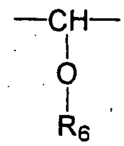

darstellt,

- $R_4$ und $R_5$ unabhängig voneinander aus der Gruppe H, Methyl gewählt werden,
- $R_6$ gewählt wird aus der Gruppe H, sowie der verzweigten und unverzweigten, gesättigten und ungesättigten Alkyl- und Acylreste mit 1 - 20 Kohlenstoffatomen.

Die Strukturformel ist nicht so zu interpretieren, daß durch den Index a alle in der Klammer repräsentierten Reste $R_4$, $R_5$ bzw $R_6$ im gesamten Molekül jeweils gleich sein müssen. Vielmehr können diese Reste in jedem der a Fragmente

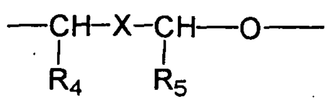

frei gewählt werden.

Eine vorteilhafte Verkörperung der vorliegenden Erfindung ist ferner die Verwendung von Wirkstoffkombinationen aus

(a) einem oder mehreren unsymmetrisch substituierten s-Triazinderivaten
(b) einer oder mehreren grenzflächenaktiven Substanzen der der allgemeinen Formel (1)

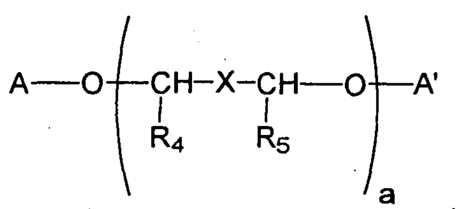

- wobei A und A' gleiche oder verschiedene organische Reste, gewählt aus der Gruppe der verzweigten und unverzweigten, gesättigten und ungesättigten Alkyl- und Acylreste und Hydroxyacylreste mit 10 - 30 Kohlenstoffatomen sowie ferner aus der Gruppe der über Esterfunktionen miteinander verbundenen Hydroxyacylgruppen, nach dem Schema

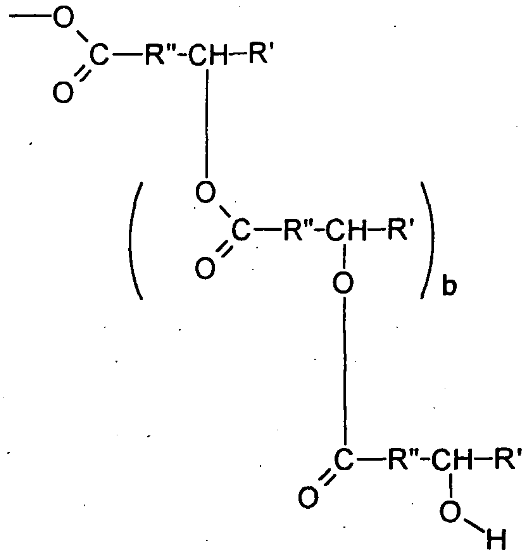

,

wobei R' gewählt wird aus der Gruppe der verzweigten und unverzweigten Alkylgruppen mit 1 bis 20 Kohlenstoffatomen und R" gewählt wird aus der Gruppe der verzweigten und unverzweigten Alkylengruppen mit 1 bis 20 Kohlenstoffatomen und b Zahlen von 0 bis 200 annehmen kann,

- a eine Zahl von 1 bis 100, vorzugsweise 2 bis 60, insbesondere 5 bis 40 darstellt,
- X eine Einfachbindung oder die Gruppe

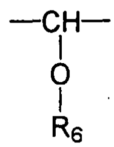

- darstellt,
- $R_4$ und $R_5$ unabhängig voneinander aus der Gruppe H, Methyl gewählt werden,
- $R_6$ gewählt wird aus der Gruppe H, sowie der verzweigten und unverzweigten, gesättigten und ungesättigten Alkyl- und Acylreste mit 1 - 20 Kohlenstoffatomen,

zum Erzielen bzw. zur Erhöhung der Stabilität von W/O-Emulsionen gegen Ausölen.

Erfindungsgemäße Zubereitungen bzw. die geschilderten erfindungsgemäßen Verwendungen helfen den geschilderten Nachteilen des Standes der Technik in überraschender Weise ab. Erfindungsgemäß sind höhere Lichtschutzfaktoren erreichbar als der Stand der Technik dies hätte annehmen lassen.

Femer ließ der Stand der Technik nicht erahnen, daß erfindungsgemäß wasserfeste Zubereitungen erhältlich sind, welche erheblich höhere Wasserfestigkeit erzielen können als Zubereitungen des Standes der Technik, wodurch auch beispielsweise nach dem Baden noch hohe Lichtschutzfaktoren erreichbar sind.

In einer vorteilhaften Ausführungsform betrifft die vorliegende Erfindung den Einsatz eines oder mehrerer unsymmetrisch substituierten s-Triazinderivate, deren chemische Struktur durch die generische Formel (2)

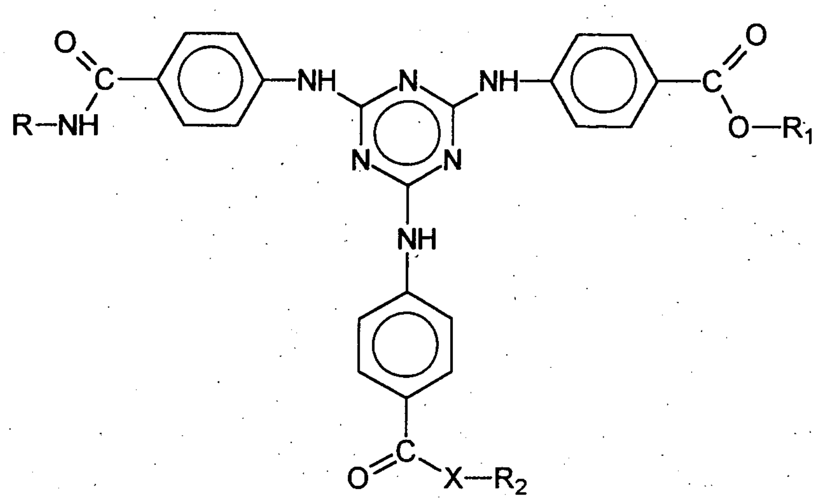

wiedergegeben wird, wobei

- R einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt,
- X ein Sauerstoffatom oder eine NH-Gruppe darstellt,
- $R_1$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$-Alkylgruppen, oder ein Wasserstoffatom, ein Alkalimetallatom, eine Ammoniumgruppe oder eine Gruppe der Formel

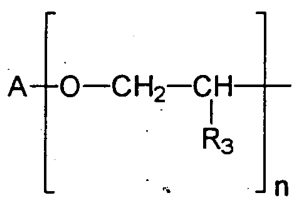

bedeutet, in welcher

- A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,
- $R_3$ ein Wasserstoffatom oder eine Methylgruppe darstellt,
- n eine Zahl von 1 bis 10 darstellt,
- $R_2$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt, wenn X die NH-Gruppe darstellt, und einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

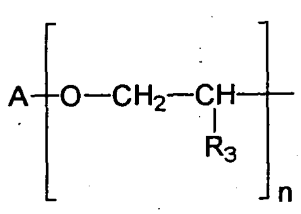

bedeutet, in welcher

- A einen verzweigten oder unverzweigten $C_1$-$C_{18}$-Alkylrest, einen $C_5$-$C_{12}$-Cycloalkyl- oder Arylrest dar-

stellt, gegebenenfalls substituiert mit einer oder mehreren $C_1$-$C_4$- Alkylgruppen,

$R_3$ ein Wasserstoffatom oder eine Methylgruppe darstellt,

n eine Zahl von 1 bis 10 darstellt,

wenn X ein Sauerstoffatom darstellt.

In einer besonders bevorzugten Ausführungsform enthalten kosmetische oder dermatologische Lichtschutzzubereitungen gemäß der vorliegenden Erfindung als unsymmetrisch substituiertes s-Triazinderivat das Dioctylbutylamidotriazon, dessen chemische Struktur durch die Formel

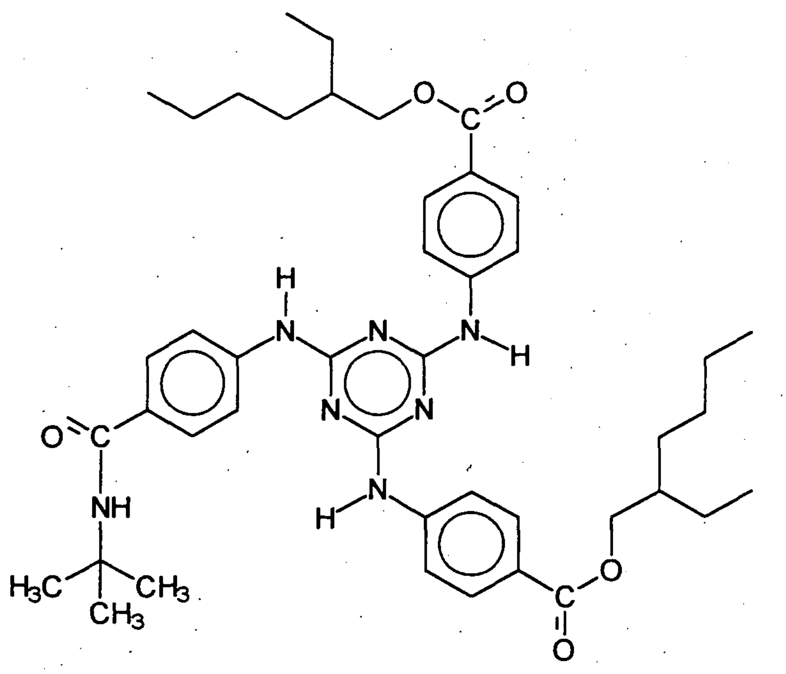

repräsentiert wird.

In den Substanzen der allgemeinen Strukturformel

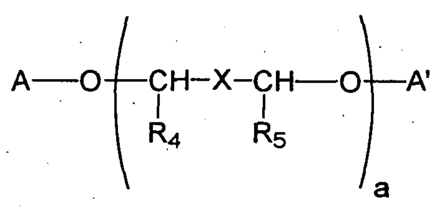

können A, A', $R_4$ und $R_5$ vorteilhaft Wasserstoffatome darstellen, werden aber ebenfalls vorteilhaft aus der Gruppe Methyl-, Ethyl-, Propyl- und Isopropyl-, Myristoyl-, Palmitoyl-, Stearoyl- und Eicosoyl- gewählt, sowie aus der Gruppe, die sich durch die chemischen Strukturen.

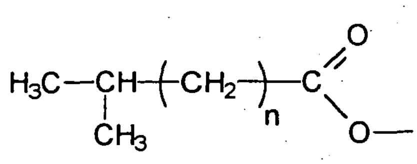

wobei n Zahlen von 1 0 bis 20 annimmt, von diesen bevorzugt der Isostearoylrest, bzw.

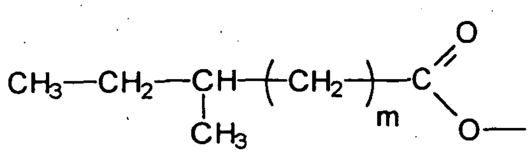

wobei m Zahlen von 9 bis 19 annimmt.

Als vorteilhaft haben sich erfindungsgemäße lichtschutzwirksame Wirkstoffkombinationen erwiesen, in welchen die grenzflächenaktiven Substanzen der der allgemeinen Formel (1) Monoglycerinester, Diglycerinester, Triglycerinester bzw. Tetraglycerinester darstellen und beispielsweise Monoester der Isostearinsäure sind, insbesondere vorteilhaft ist das Tetraglycerinmonoisostearat, welches analog zur CTFA-Nomenklatur auch Polyglyceryl-4-Iso-stearat genannt wird.

Solche Isostearinsäureester sind beispielsweise erhältlich unter der Produktbezeichnung "Isolan GI 34" der Gesellschaft Henkel Goldschmidt Chemical Corp.

Als besonders vorteilhaft haben sich auch erfindungsgemäße lichtschutzwirksame Wirkstoffkombinationen erwiesen, in welchen die grenzflächenaktiven Substanzen der der allgemeinen Formel (1) Monoglycerinester, Diglycerinester, Triglycerinester bzw. Tetraglycerinester darstellen und Diester der Isostearinsäure sind, insbesondere bevorzugt ist das Triglycerindiisostearat, welches analog zur CTFA-Nomenklatur auch Polyglyceryl-3-Diisostearat genannt wird.

Solche Isostearinsäureester sind beispielsweise erhältlich unter der Produktbezeichnung "Lameform TGI" der Gesellschaft Henkel KGaA.

Als ebenfalls besonders vorteilhaft haben sich erfindungsgemäße lichtschutzwirksame Wirkstoffkombinationen erwiesen, in welchen die grenzflächenaktiven Substanzen der der allgemeinen Formel (1) Monoglycerinester, Diglycerinester, Triglycerinester bzw. Tetraglycerinester darstellen und Gemische aus Monoestern und Diestern der Isostearinsäure enthalten, insbesondere bevorzugt dabei sind etwa äquimolare Gemische, wie beispielsweise das Diglycerinsesquiisostearat, welches analog zur CTFA-Nomenklatur auch Polyglyceryl-2-Sesquiisostearat genannt wird.

Solche Isostearinsäureester sind beispielsweise erhältlich unter der Produktbezeichnung "Hostacerin DGI" der Gesellschaft Hoechst AG.

Als besonders vorteilhaft haben sich erfindungsgemäße lichtschutzwirksame Wirkstoffkombinationen erwiesen, in welchen die grenzflächenaktiven Substanzen der der allgemeinen Formel (1) die Polyesterreste von der Hydroxystearinsäure abgeleitet sind, insbesondere vorteilhaft das "Polyglyceryl-2-Polyhydroxystearat", welches unter den Regstriernummern 156531-21-4 bzw. 144470-58-6 in den "Chemical Abstracts" abgelegt ist, und welcher beispielsweise unter der Warenbezeichnung DEHYMULS® PGPH von der Henkel KGaA erhältlich ist.

Ein Beispiel für besonders vorteilhaft im Sinne der vorliegenden Erfindung zu verwendende grenzflächenaktiven Substanzen ist ferner das PEG-30-Dipolyhydroxystearat, welches von der Gesellschaft ICI Surfactants unter der Warenbezeichnung ARLACEL® P135 verkauft wird.

Erfindungsgemäß können diese grenzflächenaktiven Substanzen der Formel (1) in Konzentrationen von 0,005 bis 50 Gew.%, bezogen auf das Gesamtgewicht der Zubereitungen, vorliegen. Dabei werden Konzentrationen von 0,5 - 10 Gew.-%, insbesondere 1,0 - 5 Gew.-%, bevorzugt.

Die Herstellung erfindungsgemäßer Zubereitungen geschieht nach den üblichen, dem Fachmanne geläufigen Regeln. Vorteilhaft liegen die erfindungsgemäßen Zubereitungen als Emulsionen, bevorzugt als W/O-Emulsionen vor. Es ist aber auch möglich und erfindungsgemäß gegebenenfalls vorteilhaft, andere Formulierungsarten zu wählen, beispielsweise Hydrodispersionen, Gele, Öle, Multiple Emulsionen, beispielsweise in Form von W/O/W- oder O/W/O-Emulsionen, wasserfreie Salben bzw. Salbengrundlagen usw.

Die Gesamtmenge an einem oder mehreren unsymmetrisch substituierten s-Triazinderivaten, insbesondere von Dioctylbutylamidotriazon, in den fertigen kosmetischen oder dermatologischen Zubereitungen wird vorteilhaft aus dem Bereich von 0,1 - 20,0 Gew.-%, bevorzugt 0,5 - 8,0 Gew.-% gewählt, bezogen auf das Gesamtgewicht der Zubereitungen.

Erfindungsgemäße kosmetische und dermatologische Zubereitungen enthalten vorteilhaft außerdem anorganische Pigmente auf Basis von Metalloxiden und/oder anderen in Wasser schwerlöslichen oder unlöslichen Metallverbindungen, insbesondere der Oxide des Titans ($TiO_2$), Zinks ($ZnO$), Eisens (z.B. $Fe_2O_3$), Zirkoniums ($ZrO_2$), Siliciums ($SiO_2$), Mangans (z.B. $MnO$), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Es ist besonders vorteilhaft im Sinne der vorliegenden Erfindung, wenngleich nicht zwingend, wenn die anorganischen Pigmente in hydrophober. Form vorliegen, d.h., daß sie oberflächlich wasserabweisend behandelt sind.

Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Rektion gemäß

$$n\ TiO_2 + m\ (RO)_3\ Si\text{-}R' \text{ -> } n\ TiO_2\ (oberfl.)$$

erzeugt wird n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen MT 100 T von der Firma TAYCA, ferner M 160 von der Firma Kemira sowie T 805 von der Firma Degussa erhältlich.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die kosmetischen und dermatologischen Zubereitungen gemäß der Erfindung können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B: pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Lipidphase kann vorteilhaft gewählt werden aus folgender Substanzgruppe:

- Mineralöle, Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkybenzoat und 2-Ethylhexylisostearat, Mischungen aus $C_{12-15}$-Alkybenzoat und Isotridecylisononanoat sowie Mischungen aus $C_{12-15}$-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln

oder in Kombination.

Die kosmetischen oder dermatologischen Lichtschutzzubereitungen enthalten vorteilhaft anorganische Pigmente, insbesondere Mikropigmente, z.B. in Mengen von 0,1 Gew.-% bis 30 Gew.-%, vorzugsweise in Mengen von 0,5 Gew.-% bis 10 Gew.-%, insbesondere aber 1 Gew.-% bis 6 Gew.-%, bezogen auf das Gesamtgewicht der Zubereitungen.

Es ist erfindungsgemäß vorteilhaft, außer den erfindungsgemäßen Kombinationen öllösliche UVA-Filter und/ oder UVB-Filter in der Lipidphase und/oder wasserlösliche UVA-Filter und/oder UVB-Filter in der wäßrigen Phase einzusetzen.

Vorteilhaft können die erfindungsgemäßen Lichtschutzformulierungen weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Die weiteren UVB-Filter können öllöslich oder wasserlöslich sein. Vorteilhafte öllösliche UVB-Filtersubstanzen sind z.B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)-ester;

Vorteilhafte wasserlösliche UVB-Filtersubstanzen sind z.B.:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate des 3-Benzylidencamphers, wie z.B. 4-(2-Oxo-3-bornylidenmethyl)-benzolsulfonsäure, 2-Methyl-5-(2-oxo-3-bornylidenmethyl)sulfonsäure und deren Salze.

Die Liste der genannten weiteren UVB-Filter, die in Kombination mit den erfindungsgemäßen Wirkstoffkombinationen verwendet werden können, soll selbstverständlich nicht limitierend sein.

Es kann auch von Vorteil sein, die erfindungsgemäßen Kombinationen mit weiteren UVA-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Beispiel 1**

| | Gew.-% |
|---|---|
| Polyglyceryl-2-Polyhydroxystearat | 5,00 |
| Dioctylbutylamidotriazon | 6,00 |
| $C_{12-15}$-Alkylbenzoate | 10,00 |
| Dicaprylylether | 3,00 |
| Mineralöl | 4,00 |
| $MgSO_4$ | 0,70 |
| Glycerin | 3,00 |

(fortgesetzt)

| | Gew.-% |
|---|---|
| Farbstoffe, Konservierungsmittel | q.s. |
| Wasser | ad 100,00 |

**Beispiel 2**

| | Gew.-% |
|---|---|
| Polyglyceryl-2-Polyhydroxystearat | 3,00 |
| Polyglyceryl-3-diisostearat | 2,00 |
| Dioctylbutylamidotriazon | 3,00 |
| Mineralöl | 10,00 |
| Isohexadecan | 4,00 |
| Capryl-/Caprinsäure-Triglyceride | 8,00 |
| 4-Methylbenzylidencampher | 2,00 |
| $TiO_2$ | 2,00 |
| $MgSO_4$ | 0,70 |
| Natrium-2-Hydroxy-4-methoxybenzophenon-5-sulfonat | 3,00 |
| NaOH | 0,50 |
| Butylenglykol | 5,00 |
| Farbstoffe, Konservierungsmittel | q.s. |
| Wasser | ad 100,00 |

**Beispiel 3**

| | Gew.-% |
|---|---|
| Hostacerin DGI | 6,00 |
| Dioctylbutylamidotriazon | 3,00 |
| Mineralöl | 10,00 |
| 2-Octyldodecanol | 5,00 |
| Dicaprylylether | 7,00 |
| t-Butylphenyl-methoxyphenylpropandion | 2,00 |
| ZnO | 2,00 |
| Konservierungsmittel | 0,50 |
| $ZnSO_4$ | 0,70 |
| Glycerin | 10,00 |
| Ethanol | 2,00 |
| Farbstoffe, Konservierungsmittel | q.s. |
| Wasser | ad 100,00 |

**Beispiel 4**

| | Gew.-% |
|---|---|
| Polyglyceryl-4-Isostearat | 2,00 |

(fortgesetzt)

| | Gew.-% |
|---|---|
| Polyglyceryl-2-Polyhydroxystearat | 4,00 |
| Ricinusöl | 3,00 |
| $C_{12-15}$-Alkylbenzoate | 5,00 |
| Mineralöl | 8,00 |
| Dioctylbutylamidotriazon | 6,00 |
| 4-Methylbenzylidencampher | 4;00 |
| t-Butylphenyl-methoxyphenylpropandion | 2,00 |
| $TiO_2$ | 2,00 |
| $MgSO_4$ | 0,70 |
| Glycerin | 5,00 |
| Farbstoffe, Konservierungsmittel | q.s. |
| Wasser | ad100,00 |

**Beispiel 5**

| | Gew.-% |
|---|---|
| Polyglyceryl-2-Polyhydroxystearat | 6,00 |
| Dioctylbutylamidotriazon | 8,00 |
| 4-Methylbenzylidencampher | 3,00 |
| t-Butylphenyl-methoxyphenylpropandion | 1,50 |
| $C_{12-15}$-Alkylbenzoate | 10,00 |
| TiO2 | 3,00 |
| Mineralöl | 5,00 |
| $MgSO_4$ | 0,70 |
| Natrium-2-Hydroxy-4-methoxybenzophenon-5-sulfonat | 4,00 |
| NaOH | 0,70 |
| Glycin | 1,00 |
| Glycerin | 3,00 |
| Farbstoffe, Konservierungsmittel | q.s. |
| Wasser | ad100,00 |

**Patentansprüche**

1. Kosmetische oder dermatologische Lichtschutzzubereitungen in Form von W/O-Emulsionen, enthaltend Wirkstoffkombinationen aus

   (a) einem oder mehreren unsymmetrisch substituierten s-Triazinderivaten
   (b) einer oder mehreren grenzflächenaktiven Substanzen der der allgemeinen Formel

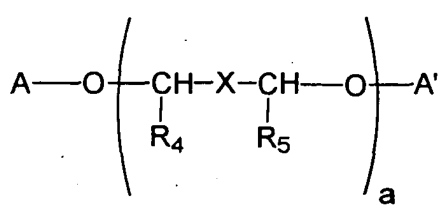

,

- wobei A und A' gleiche oder verschiedene organische Reste, gewählt aus der Gruppe der verzweigten und unverzweigten, gesättigten und ungesättigten Alkyl- und Acylreste und Hydroxyacylreste mit 10 - 30 Kohlenstoffatomen sowie ferner aus der Gruppe der über Esterfunktionen miteinander verbundenen Hydroxyacylgruppen, nach dem Schema

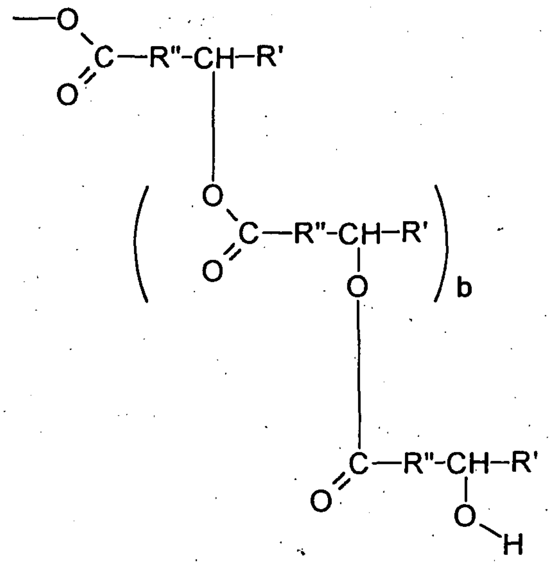

wobei R' gewählt wird aus der Gruppe der verzweigten und unverzweigten Alkylgruppen mit 1 bis 20 Kohlenstoffatomen und R" gewählt wird aus der Gruppe der verzweigten und unverzweigten Alkylengruppen mit 1 bis 20 Kohlenstoffatomen und b Zahlen von 0 bis 200 annehmen kann,

- a eine Zahl von 1 bis 100, vorzugsweise 2 bis 60, insbesondere 5 bis 40 darstellt,
- X eine Einfachbindung oder die Gruppe

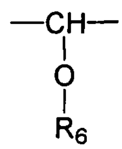

darstellt,

- $R_4$ und $R_5$ unabhängig voneinander aus der Gruppe H, Methyl gewählt werden,
- $R_6$ gewählt wird aus der Gruppe H, sowie der verzweigten und unverzweigten, gesättigten und ungesättigten Alkyl- und Acylreste mit 1 - 20 Kohlenstoffatomen.

**2.** Verwendung von Wirkstoffkombinationen aus

(a) einem oder mehreren unsymmetrisch substituierten s-Triazinderivaten
(b) einer oder mehreren grenzflächenaktiven Substanzen der der allgemeinen Formel (1)

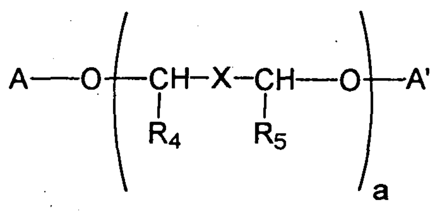

,

- wobei A und A' gleiche oder verschiedene organische Reste, gewählt aus der Gruppe der verzweigten und unverzweigten, gesättigten und ungesättigten Alkyl- und Acylreste und Hydroxyacylreste mit 10 - 30 Kohlenstoffatomen sowie ferner aus der Gruppe der über Esterfunktionen miteinander verbundenen Hydroxyacylgruppen, nach dem Schema

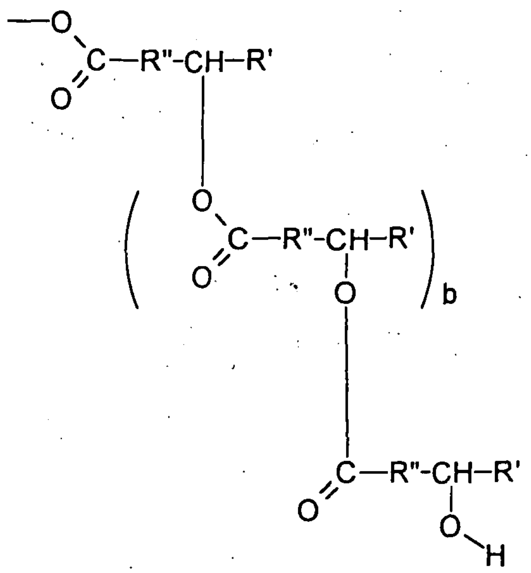

,

wobei R' gewählt wird aus der Gruppe der verzweigten und unverzweigten Alkylgruppen mit 1 bis 20 Kohlenstoffatomen und R" gewählt wird aus der Gruppe der verzweigten und unverzweigten Alkylengruppen mit 1 bis 20 Kohlenstoffatomen und b Zahlen von 0 bis 200 annehmen kann,
- a eine Zahl von 1 bis 100, vorzugsweise 2 bis 60, insbesondere 5 bis 40 darstellt,
- X eine Einfachbindung oder die Gruppe

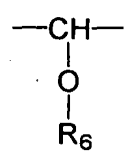

- darstellt,
- $R_4$ und $R_5$ unabhängig voneinander aus der Gruppe H, Methyl gewählt werden,
- $R_6$ gewählt wird aus der Gruppe H, sowie der verzweigten und unverzweigten, gesättigten und ungesättigten Alkyl- und Acylreste mit 1 - 20 Kohlenstoffatomen,

zum Erzielen bzw. zur Erhöhung der Stabilität von W/O-Emulsionen gegen Ausölen.

3. Lichtschutzzubereitungen nach Anspruch 1 oder Verwendung nach Anspruch 2, **dadurch gekennzeichnet, daß** das oder die unsymmetrisch substituierten s-Triazinderivate aus der Gruppe der Substanzen gewählt wird oder werden, deren chemische Struktur durch die generische Formel (2)

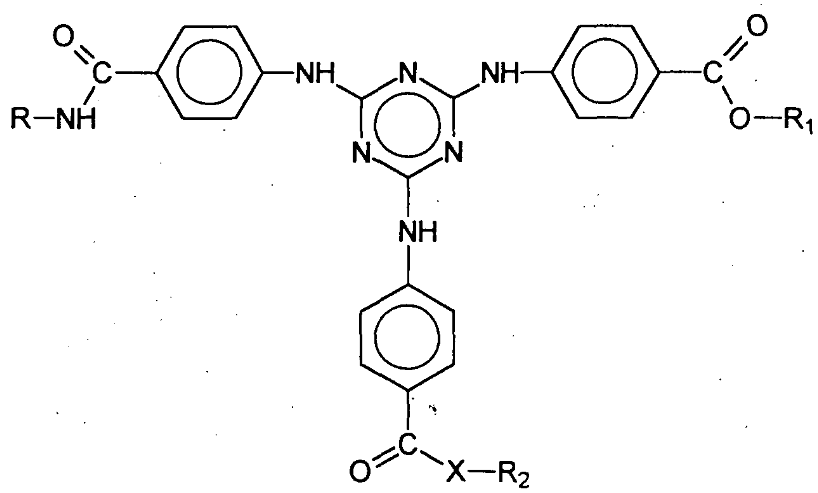

wiedergegeben wird, wobei

R einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt,
X ein Sauerstoffatom oder eine NH-Gruppe darstellt,
$R_1$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

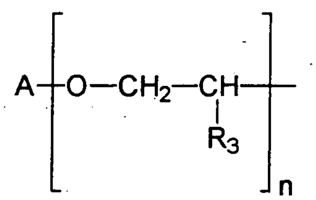

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,
$R_3$ ein Wasserstoffatom oder eine Methylgruppe darstellt,
n eine Zahl von 1 bis 10 darstellt,

$R_2$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt, wenn X die NH-Gruppe darstellt, und einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

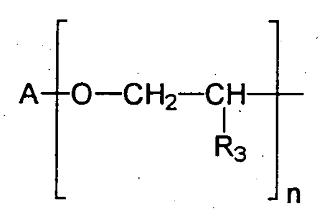

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,
$R_3$ ein Wasserstoffatom oder eine Methylgruppe darstellt,
n eine Zahl von 1 bis 10 darstellt,

wenn X ein Sauerstoffatom darstellt.

**4.** Lichtschutzzubereitungen nach Anspruch 1 oder Verwendung nach Anspruch 2, **dadurch gekennzeichnet, daß** als unsymmetrisch substituiertes Triazinderivat das Dioctylbutylamidotriazon gewählt wird.

**5.** Lichtschutzzubereitungen nach Anspruch 1 oder Verwendung nach Anspruch 2, **dadurch gekennzeichnet, daß** die die Gesamtmenge an einem oder mehreren unsymmetrisch substituierten s-Triazinderivaten, insbesondere von Dioctylbutylamidotriazon, in den fertigen kosmetischen oder dermatologischen Zubereitungen aus dem Bereich von 0,1 - 20,0 Gew.-%, bevorzugt 0,5 - 8,0 Gew.-% gewählt wird, bezogen auf das Gesamtgewicht der Zubereitungen.

**6.** Lichtschutzzubereitungen nach Anspruch 1 oder Verwendung nach Anspruch 2, **dadurch gekennzeichnet, daß** die grenzflächenaktive Substanze oder die grenzflächenaktiven Substanzen der Formel (1) gewählt werden aus der Gruppe Polyglyceryl-2-Diisostearat, Polyglyceryl-3-Diisostearat, Polyglyceryl-4-Isostearat, Polyglyceryl-2-Sesquiisostearat, Polyglyceryl-2-Polyhydroxystearat, PEG-30-Dipolyhydroxystearat.

**7.** Lichtschutzzubereitungen nach Anspruch 1 oder Verwendung nach Anspruch 2, **dadurch gekennzeichnet, daß** die grenzflächenaktive Substanz oder die grenzflächenaktiven Substanzen der Formel (1) in Konzentrationen von 0,005 bis 50 Gew.%, bezogen auf das Gesamtgewicht der Zubereitungen, bevorzugt 0,5 - 10 Gew.-%, insbesondere bevorzugt 1,0 - 5 Gew.-%, vorliegt bzw. vorliegen.